Europäisches Patentamt

European Patent Office

Office européen des brevets

(11) Numéro de publication: **0 459 901 B1**

## (12) FASCICULE DE BREVET EUROPEEN

(45) Date de publication du fascicule du brevet: **13.12.95**    (51) Int. Cl.6: **A61K 7/13**

(21) Numéro de dépôt: **91401398.2**

(22) Date de dépôt: **30.05.91**

Le dossier contient des informations techniques présentées postérieurement au dépôt de la demande et ne figurant pas dans le présent fascicule.

(54) **Procédé de teinture des fibres kératiniques avec le 2,6-diméthyl 1,4-diamino benzène et/ou le 2,3-diméthyl 1,4-diamino benzène et/ou le 2,6-diéthyl 1,4-diamino benzène en milieu acide et compositions mises en oeuvre**

(30) Priorité: **31.05.90 FR 9006802**

(43) Date de publication de la demande:
**04.12.91 Bulletin 91/49**

(45) Mention de la délivrance du brevet:
**13.12.95 Bulletin 95/50**

(84) Etats contractants désignés:
**AT BE CH DE DK ES FR GB GR IT LI NL SE**

(56) Documents cités:
FR-A- 2 615 732     GB-A- 2 018 302
GB-A- 2 018 836     GB-A- 2 026 553
GB-A- 2 066 860     GB-A- 2 205 329

(73) Titulaire: **L'OREAL**
**14, rue Royale**
**F-75008 Paris (FR)**

(72) Inventeur: **Cotteret, Jean**
**15, allée des Meuniers**
**F-78480 Verneuil-sur-Seine (FR)**

(74) Mandataire: **Casalonga, Axel et al**
**BUREAU D.A. CASALONGA - JOSSE**
**Morassistrasse 8**
**D-80469 München (DE)**

Rank Xerox (UK) Business Services
(3.10/3.09/3.3.3)

## Description

La présente invention est relative à un nouveau procédé de teinture des fibres kératiniques, en particulier des fibres kératiniques humaines, mettant en oeuvre le 2,6-diméthyl 1,4-diamino benzène et/ou le 2,3-diméthyl 1,4-diamino benzène et/ou le 2,6-diéthyl 1,4-diamino benzène en association avec un agent oxydant en milieu acide et aux compositions mises en oeuvre au cours de ce procédé.

Il est connu de teindre les fibres kératiniques et en particulier les cheveux humains avec des compositions tinctoriales contenant des précurseurs de colorants d'oxydation, en particulier des ortho ou paraphénylènediamines, des ortho ou paraaminophénols appelés généralement "bases d'oxydation".

De tels précurseurs de colorants d'oxydation, notamment les paraphénylènediamines, ont été mis en oeuvre dans les procédés de teinture décrits dans les demandes de brevet GB 2 018 302 et GB 2 026 553.

On sait également que l'on peut faire varier les nuances obtenues avec ces bases d'oxydation en les associant à des coupleurs appelés modificateurs de coloration, choisis notamment parmi les métadiamines aromatiques, les métaaminophénols et les métadiphénols.

Le coloriste des fibres kératiniques cherche généralement à obtenir des colorations ayant de bonnes résistances à la lumière, aux lavages, à la transpiration et aux intempéries, notamment par le choix des bases d'oxydation permettant d'obtenir de tels résultats.

Dans les conditions actuelles d'utilisation, les milieux de la composition oxydante et de la composition colorante sont alcalins. Or, cette alcalinité entraîne généralement une détérioration des fibres kératiniques.

Le brevet GB-A-2.018.302 décrit des procédés de teinture des fibres kératiniques à plusieurs étapes et envisage en particulier l'application sur les cheveux d'une composition à pH acide, mais cette application est suivie, sans rinçage intermédiaire, par la mise en oeuvre d'une deuxième étape comprenant l'application d'une deuxième composition ajustée à un pH tel que le pH de la composition globale au niveau des cheveux soit alcalin.

Pour éviter ou réduire cette dégradation des fibres kératiniques, on a préconisé plus récemment d'appliquer sur les fibres un mélange extemporané de l'agent oxydant et de certains précurseurs de colorants d'oxydation comme le 1,4-diamino benzène à pH acide.

Cependant, la demanderesse a constaté, notamment dans le cas du 1,4-diamino benzène, qu'un tel procédé de teinture entraînait de profondes modifications de la cinétique de la réaction et de la teinte des fibres par rapport à un même procédé de teinture effectué en milieu alcalin et ne satisfaisait plus aux exigences du coloriste.

La demanderesse vient de découvrir que l'utilisation du 2,6-diméthyl 1,4-diamino benzène et/ou du 2,3-diméthyl 1,4-diamino benzène et/ou du 2,6-diéthyl 1,4-diamino benzène, en présence d'un agent oxydant en milieu acide, conduisait à des colorations pratiquement identiques à celles obtenues dans les conditions habituelles d'utilisation et présentait par ailleurs d'une manière surprenante une plus grande ténacité à l'action de la transpiration, des shampooings, des traitements chimiques ou des agents atmosphériques tels que la lumière.

La présente invention a donc pour objet un procédé en une etape de teinture des fibres kératiniques, en particulier des fibres kératiniques humaines telles que les cheveux, comprenant l'application sur ces fibres d'une composition contenant au moins un précurseur de colorant d'oxydation encore appelé "base d'oxydation" choisi parmi le 2,6-diméthyl 1,4-diamino benzène, le 2,3-diméthyl 1,4-diamino benzène ou le 2,6-diéthyl 1,4-diamino benzène et au moins un agent oxydant, à pH acide.

D'autres objets de l'invention apparaîtront à la lecture de la description des exemples qui suivent.

Le procédé de teinture en une etape des fibres kératiniques et en particulier des fibres kératiniques humaines telles que les cheveux, conforme à l'invention, est essentiellement caractérisé par te fait que l'on applique sur ces fibres au moins une composition comprenant, dans un milieu approprié pour la teinture, un précurseur de colorant d'oxydation choisi parmi le 2,6-diméthyl 1,4-diamino benzène et/ou le 2,3-diméthyl 1,4-diamino benzène ou le 2,6-diéthyl 1,4-diamino benzène, ainsi que les sels de ces composés et un agent oxydant; le pH de la composition appliquée sur les fibres est inférieur à 7 et ladite composition ne contient pas d'ion iodure en quantité suffisante pour oxyder ledit précurseur de colorant d'oxydation.

Les précurseurs de colorants par oxydation conformes à la présente invention, peuvent être introduits dans la composition tinctoriale, soit sous forme de bases libres, soit sous forme de sels, tels que sous forme de chlorhydrates, de bromhydrates, de sulfates ou de tartrates.

L'agent oxydant est choisi de préférence parmi le peroxyde d'hydrogène, le peroxyde d'urée, les bromates de métaux alcalins, les persels tels que les perborates et persulfates. Le peroxyde d'hydrogène est particulièrement préféré.

Le pH de la composition appliquée sur les fibres kératiniques, en particulier les cheveux, a une valeur inférieure à 7 et est compris de préférence entre 3 et 6,9. Ce pH est ajusté par l'utilisation d'agents

EP 0 459 901 B1

acidifiants bien connus dans le domaine de la teinture des fibres kératiniques, et en particulier des cheveux humains, tels que des acides minéraux ou organiques comme l'acide chlorhydrique, l'acide phosphorique, les acides carboxyliques comme l'acide tartrique, l'acide citrique, les acides sulfoniques.

Les compositions définies ci-dessus appliquées dans la teinture des fibres kératiniques, peuvent également contenir en plus d'autres précurseurs de colorants d'oxydation, soit de type para, soit de type ortho tels que, par exemple : le paraaminophénol, la 2,5-diamino pyridine, la 2-hydroxy 5-amino pyridine, la 2,4,5,6-tétraamino pyrimidine, la paraphénylènediamine, la 2-chloroparaphénylènediamine, les orthoaminophénols et les orthophénylènediamines.

Les compositions de teinture conformes à la présente invention, peuvent également contenir des coupleurs connus en eux-mêmes tels que des méta diphénols, des métaaminophénols, des métaphénylène diamines, des méta N-acylaminophénols, des méta-uréidophénols, des métacarbalcoxyaminophénols, l'α-naphtol, les orthodiphénols, des coupleurs possédant un groupement méthylène actif, tels que les composés dicétoniques, les pyrazolones.

On peut citer parmi eux, en particulier, le 2,4-diamino 1,3-diméthoxybenzène, le 2,4-dihydroxy phénoxyéthanol, le 2,4-dihydroxyanisole, le métaaminophénol, la résorcine, le monométhyléther de résorcine, la 2-méthylrésorcine, le 2-méthyl 5-N-($\beta$-hydroxyéthyl) aminophénol le 2-méthyl 5-N-($\beta$-mésylaminoéthyl) aminophénol, la 6-hydroxybenzomorpholine, le 2,4-diaminoanisole, le 2,4-diaminophénoxyéthanol, la 6-aminobenzomorpholine, le 2-[N-($\beta$-hydroxyéthyl) amino]-4-amino -phénoxyéthanol, le 2-amino 4-N-($\beta$-hy-droxyéthyl)aminoanisole, le (2,4-diamino)phényl-$\beta$,$\gamma$-dihydroxypropyléther, la 2,4-diaminophénoxyéthylamine, le 2-méthyl 5-aminophénol, le 2,6-diméthyl 3-amino phénol, le 3,4-méthylènedioxyphénol, la 3,4-méthylène dioxyaniline et leurs sels. On utilise de préférence le 2,6-diméthoxy 1,3-diamino benzène, le métaaminophénol, le 2-méthyl 5-N-($\beta$-hydroxyéthylamino)phénol et l'α-naphtol.

Ces compositions peuvent également contenir des agents tensio-actifs anioniques, cationiques, non-ioniques, amphotères ou leurs mélanges.

Parmi ces agents tensio-actifs, on peut citer les alkylbenzènesulfonates, les alkylnaphtalène sulfonates, les sulfates, les éthersulfates et les sulfonates d'alcools gras, les sels d'ammonium quaternaires tels que le bromure de triméthylcétylammonium, le bromure de cétylpyridinium, les éthanolamides d'acides gras éventuellement oxyéthylénés, les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les compositions tinctoriales sont généralement aqueuses, mais elles peuvent également contenir des solvants organiques pour solubiliser des composés quine seraient par suffisamment solubles dans l'eau.

Parmi ces solvants, on peut citer à titre d'exemple, les alcanols inférieurs en $C_2$-$C_4$ tels que l'éthanol et l'isopropanol, le glycérol, les glycols ou éthers de glycols comme le 2-butoxy éthanol, l'éthylène glycol, le propylèneglycol, le monoéthyléther et le monométhyléther du diéthylèneglycol, ainsi que les alcools aromatiques comme l'alcool benzylique ou le phénoxyéthanol, ou les mélanges de ces solvants.

Les compositions appliquées sur les fibres kératiniques conformes à la présente invention, peuvent également renfermer des agents épaississants choisis en particulier parmi l'alginate de sodium, la gomme arabique, les dérivés de cellulose tels que la méthyl cellulose, l'hydroxyéthylcellulose, l'hydroxypropylcellulose, l'hydroxyméthylcellulose, la carboxyméthly cellulose, les polymères d'acide acrylique éventuellement réticulés, la gomme de xanthane. On peut également utiliser des agents épaississants minéraux tels que la bentonite.

La composition peut également renfermer des agents antioxydants choisis en particulier parmi le sulfite de sodium, l'acide thioglycolique, le bisulfite de sodium, l'acide ascorbique et l'hydroquinone, ainsi que d'autres adjuvants cosmétiquement acceptables lorsque la composition est destinée à être utilisée pour la teinture des fibres kératiniques humaines, tels que des agents de pénétration, des agents séquestrants, des conservateurs, des tampons, des parfums, etc...

La composition appliquée sur les cheveux peut se présenter sous des formes diverses, telles que de liquides, de crèmes, de gels ou sous toute autre forme appropriée pour réaliser une teinture capillaire. Elle peut être conditionnée en flacon aérosol en présence d'un agent propulseur.

Selon une forme de réalisation préférée, le procédé comporte une étape préliminaire consistant à stocker sous forme séparés, d'une part, la composition contenant dans un milieu approprié pour la teinture le 2,6-diméthyl 1,4-diamino benzène et/ou le 2,3-diméthyl 1,4-diamino benzène et/ou le 2,6-diéthyl 1,4-diamino benzène, sous forme d'un composant (A) et, d'autre part, une composition renfermant l'agent oxydant telle que définie ci-dessus, sous forme d'un composant (B), et à procéder à leur mélange extemporané avant d'appliquer ce mélange sur les fibres kératiniques, comme indiqué ci-dessus, l'application étant suivie par un temps de pose, d'un rinçage final éventuellement suivi d'un shampooing. Le composant (A) ne contient pas d'ion iodure en quantité suffisante pour oxyder ledit précurseur de colorant d'oxydation.

3

La composition appliquée sur les fibres kératiniques résulte en particulier d'un mélange de 10 à 90% du composant (A) avec 90 à 10% du composant (B) contenant un agent oxydant.

Le composant (A) qui renferme au moins le précurseur de colorant d'oxydation, a un pH compris entre 3 et 10,5 et peut être ajusté à la valeur choisie au moyen a' agents alcalinisants habituellement utilisés en teinture des fibres kératiniques, tels que l'ammoniaque, les carbonates alcalins, les alcanolamines telles que les mono-, di- et triéthanolamines, ainsi que leurs dérivés ou des agents acidifiants classiques, tels que les acides minéraux ou organiques comme les acides chlorhydrique, phosphorique, les acides carboxyliques comme les acides tartrique, citrique, les acides sulfoniques.

Cette composition peut renfermer les différents autres adjuvants mentionnés ci-dessus, notamment les précurseurs de colorants d'oxydation ortho ou para ou les coupleurs.

Les précurseurs de colorants par oxydation du type para ou ortho, autres que le 2,6-diméthyl 1,4-diamino benzène, le 2,3-diméthyl 1,4-diamino benzène et le 2,6-diéthyl 1,4-diamino benzène, sont présents dans des proportions comprises entre 0,02 et 10% en poids et de préférence entre 0,05 et 5% en poids par rapport au poids total du composant (A).

Les coupleurs tels que mentionnés ci-dessus sont présents dans des concentrations comprises entre 0,02 et 10% en poids et de préférence entre 0,05 et 5% en poids par rapport au poids total du composant (A).

La concentration en 2,6-diméthyl 1,4-diamino benzène et/ou en 2,3-diméthyl 1,4-diamino benzène et/ou en 2,6-diéthyl 1,4-diaminobenzène est comprise entre 0,02 et 10% en poids et de préférence entre 0,05 et 5% en poids par rapport au poids total du composant (A).

Les agents tensio-actifs sont présents dans le composant (A) dans des proportions de 0,1 à 55% en poids.

Les agents solvants éventuellement présents en plus de l'eau sont présents dans des proportions comprises entre 0,5 et 40% en poids et en particulier entre 5 et 30% en poids par rapport au poids total du composant (A).

Les agents épaississants sont présents de préférence dans des proportions comprises entre 0,1 et 5%, et en particulier entre 0,2 et 3% en poids.

Les agents antioxydants mentionnés ci-dessus sont de préférence présents dans le composant (A) dans des proportions comprises entre 0,02 et 1,5% en poids par rapport au poids total du composant (A).

Le composant (A) conforme à l'invention, peut se présenter sous des formes diverses, telles que des liquides, des crèmes, des gels ou sous toute autre forme appropriée pour réaliser une teinture capillaire. Il peut être conditionné en flacon aérosol en présence d'un agent propulseur.

Le composant (B) renfermant l'agent oxydant tel que défini ci-dessus a un pH inférieur à 7. Ce pH peut avoir une valeur minimum de 1, et de préférence il est compris entre 1,5 et 3,5. Ce composant (B) peut être acidifié avec le même type d'agents acidifiants que ceux utilisés pour le composant (A).

Il peut se présenter sous forme de liquides plus ou moins épaissis, de laits ou de gels.

L'agent de teinture à deux composants conforme à la présente invention, peut être conditionné dans un dispositif à plusieurs compartiments ou kit de teinture, ou tout autre système de conditionnement à plusieurs compartiments, dont l'un des compartiments renferme le composant (A) et le second compartiment renferme le composant (B); ces dispositifs pouvant être équipés d'un moyen permettant de délivrer sur les cheveux le mélange souhaité, tel que le dispositif décrit dans le brevet US-A-4 823 985 de la demanderesse.

Conformément à l'invention, le procédé de teinture consiste à appliquer sur les cheveux le mélange obtenu, à le laisser poser pendant 3 à 40 minutes, puis à rincer les cheveux et éventuellement effectuer un shampooing.

Les exemples suivants sont destinés à illustrer l'invention sans pour autant présenter un caractère limitatif.

EXEMPLES 1 à 10

On procède à la teinture des cheveux en appliquant sur des cheveux permanentés gris à 90% de blancs un mélange extemporané de la composition colorante (A) et de la composition oxydante (B).

Ce mélange présente le pH indiqué dans le tableau des exemples qui suivent.

On laisse agir ce mélange pendant 30 minutes, puis on rince les cheveux, on effectue un shampooing.

# EP 0 459 901 B1

Après séchage, les cheveux sont teints dans la nuance précisée au bas du tableau.

| en g | 1 | 2 | 3 | 4 | 5 | 6 |
|---|---|---|---|---|---|---|
| A) Composition colorante | | | | | | |
| 2,6-diméthyl 1,4-diaminobenzène, 2 HCl | 0,627 | | | 0,657 | 0,657 | |
| 2,3-diméthyl 1,4-diaminobenzène, 2 HCl | | 0,627 | 0,627 | | | 0,627 |
| 2-méthyl 5-N-(β-hydroxyéthyl) aminophénol | | 0,501 | | 0,417 | | 0,417 |
| α-naphtol | 0,327 | | 0,327 | | 0,327 | |
| Monoéthanolamine qs pH | 9,8 | 9,8 | 9,8 | 8,8 | 8,8 | 8,9 |
| Support 1 | X | X | X | | | |
| Support 2 | | | | X | X | X |
| Eau qsp | 100 | 100 | 100 | 100 | 100 | 100 |

| | 1 | 2 | 3 | 4 | 5 | 6 |
|---|---|---|---|---|---|---|
| B) Composition oxydante | | | | | | |
| Solution d'eau oxygénée à 20 volumes | | | | | | |
| Acide phosphorique qs pH | 1,5 | 1,5 | 1,5 | 1,5 | 1,5 | 1,5 |
| pH mélange p/p A + B | 5,5 | 5,5 | 5,5 | 6,0 | 6,4 | 6,0 |
| Nuances obtenues : | blond clair | blond irisé | blond clair cendré mat | pourpre gri-sâtre foncé | olive gri-sâtre | irisé violine |

5

|  | 7 | 8 | 9 | 10 |
|---|---|---|---|---|
| A) <u>Composition colorante</u> |  |  |  |  |
| 2,6-diméthyl 1,4-diaminobenzène, 2 HCl |  | 0,438 | 0,876 | 0,583 |
| 2,3-diméthyl 1,4-diaminobenzène, 2 HCl | 0,627 |  |  |  |
| 2-méthyl 5-N-(β-hydroxyéthyl) aminophénol |  | 0,324 |  | 0,216 |
| Métaaminophénol |  |  | 0,576 | 0,192 |
| α-naphtol | 0,327 |  |  |  |
| Monoéthanolamine  qs  pH | 8,8 | 9,1 | 8,8 | 8,9 |
| Support 2 | X | X | X | X |
| Eau    qsp | 100 | 100 | 100 | 100 |

|  | 7 | 8 | 9 | 10 |
|---|---|---|---|---|
| B) <u>Composition oxydante</u><br><br>Solution d'eau oxygénée à 20 volumes |  |  |  |  |
| Acide phosphorique  qs  pH | 1,5 | 1,1 | 1,5 | 1,1 |
| pH mélange p/p A + B | 6,1 | 6,6 |  | 6,5 |
| pH mélange 1/3 A + 2/3 B |  |  | 5,8 |  |
| <u>Nuances obtenues</u> : | blond jaunâtre grisâtre | irisé vidine | gris foncé bleuté | acajou légèrement cendré |

6

EXEMPLES 11 et 12

## COMPOSITION

| | | |
|---|---|---|
| – Colorant | X | g |
| – Nonylphénol à 4 moles d'oxyde d'éthylène, vendu sous la dénomination "SINNOPAL NP4" par la Société HENKEL | 25,5 | g |
| – Nonylphénol à 9 moles d'oxyde d'éthylène, vendu sous la dénomination "SINNOPAL NP9" par la Société HENKEL | 17,5 | g |
| – Butyléther d'éthylèneglycol | 7,0 | g |
| – Propylèneglycol | 11,0 | g |
| – Alcool éthylique | 2,0 | g |
| – Lauryléthersulfate de monoéthanolamine, vendu sous la dénomination "SACTIPON 2 OM 29" par la Société LEVER à 28% de MA | 17,9 | g |
| – Acétate de sodium | 0,8 | g |
| – Antioxydant, séquestrant | qs | |
| – Eau déminéralisée | qsp 100,0 | g |

## MODE OPERATOIRE

La composition précedemment décrite est mélangée, poids pour poids, avec de l'eau oxygénée à 20 volumes et ajustée à pH = 6,7 avec de l'acide orthophosphorique.

La préparation est appliquée sur des cheveux gris à 90% de blancs, à raison de 28 g du mélange pour 3 g de cheveux, pendant 30 minutes. Les mèches sont ensuite rincées, lavées avec un shampooing, rincées puis séchées.

7

Les cheveux sont teints dans la nuance précisée au bas du tableau :

| en g | Exemple 11 | Exemple 12 |
|---|---|---|
| 2,6-diéthyl 1,4-diaminobenzène | O,711 | O,711 |
| 6-hydroxybenzomorpholine | O,453 | |
| 2,4-diaminophénoxyéthanol,2HCl | | O,723 |
| Nuances obtenues : | Jaune mat | gris moyen bleuté |

EXEMPLES 13 et 14

On procède à la teinture des cheveux en appliquant sur des cheveux permanentés gris à 90% de blancs, un mélange extemporané de la composition colorante (A) et de la composition oxydante (B).

Ce mélange présente le pH indiqué dans le tableau des exemples qui suivent.

On laisse agir ce mélange pendant 30 minutes, puis on rince les cheveux, on effectue un shampooing. Après séchage, les cheveux sont teints dans la nuance précisée au bas du tableau ci-après.

8

| en g | 13 | 14 |
|---|---|---|
| **A) Composition colorante** | | |
| 2,6-diméthyl 1,4-diaminobenzène, 2HCl | 0,836 | 0,836 |
| 2-méthyl résorcine | | 0,248 |
| 2-méthyl 5-N-(ß-hydroxyéthyl) aminophénol | 0,19 | |
| 2-chloroparaphénylènediamine sulfate | | 0,48 |
| 6-hydroxybenzomorpholine · | 0,464 | |
| Monoéthanolamine qs pH | 9,9 | 9,8 |
| Support 3 | X | X |
| Eau qsp | 100 | 100 |
| **B) Composition oxydante** | | |
| Solution d'eau oxygénée à 20 volumes | | |
| Acide orthophosphorique qs pH | 1,2 | 1,2 |
| pH mélange p/p A+B | 6,6 | 6,3 |
| Nuances obtenues : | châtain clair doré légèrement mat | blond foncé doré irisé |

## SUPPORT DE COLORATION 1

- Nonylphénol à 4 moles d'oxyde
  d'éthylène, vendu sous la
  dénomination "SINNOPAL NP4" par
  la Société HENKEL                           25,5    g
- Nonylphénol à 9 moles d'oxyde
  d'éthylène, vendu sous la
  dénomination "SINNOPAL NP9" par
  la Société HENKEL                           17,5    g
- Butyléther d'éthylèneglycol                  7,0    g
- Propylèneglycol                       .     11,0    g
- Alcool éthylique                             2,0    g
- Lauryl éther sulfate de
  monoéthanolamine, vendu sous la
  dénomination "SACTIPON 2 OM 29"
  par la Société LEVER à 28% de MA            5,0    g MA
- Métabisulfite de sodium en
  solution aqueuse à 35% de MA               0,45   g MA
- Acétate de sodium                          0,8    g
- Antioxydant, séquestrant    qs

## SUPPORT DE COLORATION 2

| | |
|---|---|
| - Alcool oléique polyglycérolé à 2 moles de glycérol | 4,0 g |
| - Alcool oléique polyglycérolé à 4 moles de glycérol à 78% de MA | 5,69 g MA |
| - Acide oléique | 3,0 g |
| - Amine oléique à 2 moles d'oxyde d'éthylène, vendue sous la dénomination "ETHOMEEN O 12" par la Société AKZO | 7,0 g |
| - Laurylamino succinamate de diéthylaminopropyle, sel de sodium, à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de MA | 0,45 g MA |
| - Acétate d'ammonium | 0,8 g |
| - Antioxydant, séquestrant | qs |

## SUPPORT DE COLORATION 3

| | |
|---|---|
| - Alcool cétylstéarylique | 18,0 g |
| - 2-octyl dodécanol | 3,0 g |
| - Alcool cétylstéarylique oxyéthylénée à 15 moles d'oxyde d'éthylène | 3,0 g |
| - Laurylsulfate d'ammonium à 30% de MA | 3,6 g MA |
| - Thiolactate d'ammonium à 50% de MA | 0,4 g MA |

11

EP 0 459 901 B1

**Revendications**

1. Procédé de teinture en une etape des fibres kératiniques, en particulier des fibres kératiniques humaines telles que les cheveux, caractérisé par le fait que l'on applique sur ces fibres une composition contenant dans un milieu approprié pour la teinture, au moins :
   - un précurseur de colorant d'oxydation choisi parmi le 2,6-diméthyl 1,4-diamino benzène et/ou le 2,3-diméthyl 1,4-diamino benzène et/ou le 2,6-diéthyl 1,4-diamino benzène, ainsi que leurs sels;
   - au moins un agent oxydant;
   le pH de la composition appliquée sur les fibres étant inférieur à 7 et ladite composition ne contenant pas d'ion iodure en quantité suffisante pour oxyder le précurseur de colorant d'oxydation.

2. Procédé de teinture selon la revendication 1, caractérisé par le fait que l'agent oxydant est choisi parmi le peroxyde d'hydrogène, le peroxyde d'urée, les bromates de métaux alcalins, les persels.

3. Procédé selon la revendication 1 ou 2, caractérisé par le fait que le pH de la composition appliquée sur les fibres kératiniques est compris entre 3 et 6,9.

4. Procédé selon l'une quelconque des revendications 1 à 3, caractérisé par le fait que la composition utilisée pour la teinture des fibres kératiniques contient en plus d'autres précurseurs de colorants d'oxydation de type para ou ortho.

5. Procédé selon la revendication 4, caractérisé par le fait que les précurseurs de colorants par oxydation sont choisis parmi le p-aminophénol, la 2,5-diamino pyridine, la 2-hydroxy 5-amino pyridine, la 2,4,5,6-tétraamino pyrimidine, la paraphénylènediamine, la 2-chloroparaphénylènediamine, les orthoaminophénols, les orthophénylènediamines.

6. Procédé selon l'une quelconque des revendications 1 à 5, caractérisé par le fait que la composition comprend en plus des coupleurs choisis parmi les métadiphénols, les métaaminophénols, les métauréidophénols, les métaphénylènediamines, les métaacylaminophénols, les métacarbalcoxyaminophénols, l'α-naphtol, tes orthodiphénols, les coupleurs possédant un groupement méthylène actif choisis parmi les composés dicétoniques et les pyrazolones.

7. Procédé selon la revendication 6, caractérisé par le fait que les coupleurs sont choisis parmi le 2,4-diamino 1,3-diméthoxybenzène, le 2,4-dihydroxyphénoxyéthanol, le 2,4-dihydroxyanisole, le métaaminophénol, la résorcine, le monométhyléther de résorcine, la 2-méthylrésorcine, le 2-méthyl 5-N-(β-hydroxyéthyl)aminophénol, le 2-méthyl 5-N-(β-mésylaminoéthyl)aminophénol, la 6-hydroxybenzomorpholine, le 2,4-diaminoanisole, le 2,4-diaminophénoxyéthanol, la 6-aminobenzomorpholine, le 2-[N-(β-hydroxyéthyl)amino]-4-amino -phénoxyéthanol, le 2-amino 4-N-(β-hydroxyéthyl)aminoanisole, le (2,4-diamino)phényl-β, γ -dihydroxypropyléther, la 2,4-diaminophénoxyéthylamine, le 2-méthyl 5-aminophénol, le 2,6-diméthyl 3-aminophénol, le 3,4-méthylènedioxyphénol, la 3,4-méthylène dioxyaniline, et leurs sels.

8. Procédé selon l'une quelconque des revendications 1 à 7, caractérisé par le fait que la composition contient des agents tensio-actifs anioniques, cationiques, non-ioniques, amphotères ou leurs mélanges, des agents antioxydants, des agents épaississants et/ou tout autre adjuvant cosmétiquement acceptable.

9. Procédé selon l'une quelconque des revendications 1 à 8, caractérisé par le fait que le milieu approprié pour la teinture est constitué par de l'eau ou un mélange' d'eau et d'un solvant choisi parmi les alcanols inférieurs en $C_2$-$C_4$, le glycérol, les glycols ou éthers de glycols, le monoéthyléther et monométhyléther de diéthylèneglycol, les alcools aromatiques ou leurs mélanges.

10. Procédé de teinture des fibres kératiniques et en particulier des cheveux, caractérisé par le fait qu'il comporte une première étape consistant à stocker sous forme séparée les composants de l'agent de teinture comprenant
    - un composant (A) constitué par une composition contenant dans un milieu approprié pour la teinture, le 2,6-diméthyl 1,4-diamino benzène et/ou le 2,3-diméthyl 1,4-diamino benzène et/ou le 2,6-diéthyl 1,4-diamino benzène, ledit composant (A) ne contenant pas d'ion iodure en quantité

12

suffisante pour oxyder le précurseur de colorant d'oxydation; et

- un composant (B) constitué par une composition contenant dans un milieu approprié pour la teinture, un agent oxydant et à procéder avant application au mélange des compositions (A) et (B) dans des proportions de 10 à 90% pour le composant (A) et de 90 à 10% pour le composant (B), de façon à obtenir une compositionayant un pH inférieur à 7 et d'appliquer ce mélange immédiatement après préparation sur les fibres kératiniques, l'application étant suivie par un temps de pose et d'un rinçage final éventuellement suvi d'un shampooing.

11. Procédé de teinture selon l'une quelconque des revendications 1 à 9, caractérisé par le fait qu'on applique sur les cheveux la composition et qu'on la laisse poser pendant 3 à 40 minutes, que l'on rince les cheveux et qu'on procède éventuellement à un shampooing avant un nouveau rinçage et séchage.

## Claims

1. One-step process for dyeing keratinous fibres, especially human keratinous fibres such as the hair, characterised in that a composition containing, in a medium suitable for dyeing, at least:
   - one oxidation dye precursor selected from 2,6-dimethyl-1,4-diaminobenzene and/or 2,3-dimethyl-1,4-diaminobenzene and/or 2,6-diethyl-1,4-diaminobenzene, as well as their salts; and
   - at least one oxidising agent;

   is applied to these fibres, the pH of the composition applied to the fibres being less than 7 and the said composition not containing iodide ions in a sufficient quantity to oxidise the oxidation dye precursor.

2. Dyeing process according to Claim 1, characterised in that the oxidising agent is selected from hydrogen peroxide, urea peroxide, alkali metal bromates and persalts.

3. Process according to Claim 1 or 2, characterised in that the pH of the composition applied to the keratinous fibres is between 3 and 6.9.

4. Process according to any one of Claims 1 to 3, characterised in that the composition used for dyeing the keratinous fibres contains, in addition, other oxidation dye precursors of the para or ortho type.

5. Process according to Claim 4, characterised in that the oxidation dye precursors are selected from p-aminophenol, 2,5-diaminopyridine, 2-hydroxy-5-aminopyridine, 2,4,5,6-tetraaminopyrimidine, para-phenylenediamine, 2-chloro-para-phenylenediamine, ortho-aminophenols and ortho-phenylenediamines.

6. Process according to any one of Claims 1 to 5, characterised in that the composition comprises, in addition, couplers selected from meta-diphenols, meta-aminophenols, meta-ureidophenols, meta-phenylenediamines, meta-acylaminophenols, meta-carbalkoxyaminophenols, $\alpha$-naphthol, ortho-diphenols and couplers possessing an active methylene group selected from diketo compounds and pyrazolones.

7. Process according to Claim 6, characterised in that the couplers are selected from 2,4-diamino-1,3-dimethoxybenzene, 2,4-dihydroxyphenoxyethanol, 2,4-dihydroxyanisole, meta-aminophenol, resorcinol, resorcinol monomethyl ether, 2-methylresorcinol, 2-methyl-5-[N-($\beta$-hydroxyethyl)amino]phenol, 2-meth-yl-5-[N-($\beta$-mesylaminoethyl)amino]phenol, 6-hydroxybenzomorpholine, 2,4-diaminoanisole, 2,4-diaminophenoxyethanol, 6-aminobenzomorpholine, 2-[N-($\beta$-hydroxyethyl)amino]-4-aminophenoxyethanol, 2-amino-4-[N-($\beta$-hydroxyethyl)amino]anisole, 2,4-diaminophenyl $\beta,\gamma$-dihydroxypropyl ether, 2,4-diaminophenoxyethylamine, 2-methyl-5-aminophenol, 2,6-dimethyl-3-aminophenol, 3,4-methylenedioxyphenol, 3,4-methylenedioxyaniline and their salts.

8. Process according to any one of Claims 1 to 7, characterised in that the composition contains anionic, cationic, nonionic or amphoteric surfactants or mixtures thereof, antioxidants, thickening agents and/or any other cosmetically acceptable adjuvant.

9. Process according to any one of Claims 1 to 8, characterised in that the medium suitable for dyeing consists of water or a mixture of water and a solvent selected from $C_2$-$C_4$ lower alkanols, glycerol, glycols or glycol ethers, diethylene glycol monoethyl ether and monomethyl ether, aromatic alcohols or mixtures thereof.

10. Process for dyeing keratinous fibres, and especially the hair, characterised in that it entails a first step consisting in storing separately the components of the dyeing agent comprising

- a component (A) consisting of a composition containing, in a medium suitable for dyeing, 2,6-dimethyl-1,4-diaminobenzene and/or 2,3-dimethyl-1,4-diaminobenzene and/or 2,6-diethyl-1,4-diaminobenzene, the said component (A) not containing iodide ions in a sufficient quantity to oxidise the oxidation dye precursor; and
- a component (B) consisting of a composition containing an oxidising agent in a medium suitable for dyeing, and in mixing the compositions (A) and (B) in proportions of 10 to 90% for the component (A) and 90 to 10% for the component (B) before application so as to obtain a composition having a pH of less than 7, and applying this mixture to the keratinous fibres immediately after preparation, the application being followed by a period of exposure and a final rinse, optionally followed by shampooing.

11. Dyeing process according to any one of Claims 1 to 9, characterised in that the composition is applied to the hair and in that it is left in place for 3 to 40 minutes, in that the hair is rinsed and in that it is optionally shampooed before a further rinse and drying.

**Patentansprüche**

1. Verfahren in 1 Stufe zur Färbung keratinischer, insbesondere menschlicher keratinischer Fasern wie der Haare,
dadurch **gekennzeichnet,** daß
man auf diese Fasern eine Zusammensetzung, die in einem zur Färbung geeigneten Milieu mindestens:

- eine Oxidationsfarbstoff-Vorstufe enthält, die aus 2,6-Dimethyl-1,4-diaminobenzol und/oder 2,3-Dimethyl-1,4-diaminobenzol und/oder 2,6-Diethyl-1,4-diaminobenzol sowie aus deren Salzen ausgewählt ist, und
- mindestens ein oxidierendes Agens aufbringt,

wobei der pH-Wert der auf die Fasern aufgebrachten Zusammensetzung weniger als 7 beträgt und die genannte Zusammensetzung kein Jodidion in einer zum Oxidieren der Oxidationsfarbstoff-Vorstufe ausreichenden Menge enthält.

2. Verfahren zur Färbung gemäß Anspruch 1,
dadurch **gekennzeichnet,** daß
das oxidierende Agens aus Wasserstoffperoxid, Harnstoffperoxid, Alkalibromaten und Persalzen ausgewählt ist.

3. Verfahren gemäß Anspruch 1 oder 2,
dadurch **gekennzeichnet,** daß
der pH-Wert der auf die keratinischen Fasern aufgebrachten Zusammensetzung 3 bis 6,9 beträgt.

4. Verfahren gemäß jedem der Ansprüche 1 bis 3,
dadurch **gekennzeichnet,** daß
die zur Färbung der keratinischen Fasern verwendete Zusammensetzung zusätzlich weitere Oxidationsfarbstoff-Vorstufen vom para- oder ortho-Typ enthält.

5. Verfahren gemäß Anspruch 4,
dadurch **gekennzeichnet,** daß
die Oxidationsfarbstoff-Vorstufen aus p-Aminophenol, 2,5-Diaminopyridin, 2-Hydroxy-5-aminopyridin, 2,4,5,6-Tetraaminopyrimidin, p-Phenylendiamin, 2-Chlor-p-phenylendiamin, o-Aminophenolen und o-Phenylendiaminen ausgewählt sind.

6. Verfahren gemäß jedem der Ansprüche 1 bis 5,
dadurch **gekennzeichnet,** daß
die Zusammensetzung zusätzlich Kuppler enthält, die aus m-Diphenolen, m-Aminophenolen, m-Ureidophenolen, m-Phenylendiaminen, m-Acylaminophenolen, m-Carbalkoxyaminophenolen, $\alpha$-Naphthol, o-Diphenolen, aus Kupplern mit einer aktiven Methylengruppe aus Diketon-Verbindungen und Pyrazolonen ausgewählt sind.

7. Verfahren gemäß Anspruch 6,
dadurch **gekennzeichnet,** daß
die Kuppler aus 2,4-Diamino-1,3-dimethoxybenzol, 2,4-Dihydroxyphenoxyethanol,2,4-Dihydroxyanisol, m-Aminophenol, Resorcin, den Monomethylether von Resorcin, 2-Methylresorcin, 2-Methyl-5-N-($\beta$-hydroxyethyl)aminophenol, 2-Methyl-5-N-($\beta$-mesylaminoethyl)aminophenol, 6-Hydroxybenzomorpholin, 2,4-Diaminoanisol, 2,4-Diaminophenoxyethanol, 6-Aminobenzomorpholin, 2-(N-($\beta$-Hydroxyethyl)amino)-4-aminophenoxyethanol, 2-Amino-4-N-($\beta$-hydroxyethyl)aminoanisol, (2,4-Diamino)phenyl-$\beta,\gamma$-dihydroxypropylether, 2,4-Diaminophenoxyethylamin, 2-Methyl-5-aminophenol, 2,6-Dimethyl-3-aminophenol, 3,4-Methylendioxyphenol, 3,4-Methylendioxyanilin und aus deren Salzen ausgewählt sind.

8. Verfahren gemäß jedem der Ansprüche 1 bis 7,
dadurch **gekennzeichnet,** daß
die Zusammensetzung anionische, kationische, nicht-ionische oder amphotere oberflächenaktive Mittel oder deren Mischungen, Antioxidantien, Verdickungsmittel und/oder jeden weiteren kosmetisch geeigneten Hilfsstoff enthält.

9. Verfahren gemäß jedem der Ansprüche 1 bis 8,
dadurch **gekennzeichnet,** daß
das zur Färbung geeignete Milieu aus Wasser oder einer Mischung aus Wasser und einem Lösungsmittel zusammengesetzt ist, das aus $C_{2-4}$-Niedrigalkanolen, Glycerin, Glycolen oder Ethern von Glycolen, aus Monoethylether und Monomethylether von Diethylenglycol, aus aromatischen Alkoholen oder deren Mischungen ausgewählt ist.

10. Verfahren zur Färbung keratinischer Fasern und insbesondere der Haare,
dadurch **gekennzeichnet,** daß
es eine Vorstufe umfaßt, wobei man in getrennter Form die Bestandteile des Färbemittels aufbewahrt und vorhält, welches
- einen Bestandteil (A) aus einer Zusammensetzung, die in einem zur Färbung geeigneten Milieu 2,6-Dimethyl-1,4-diaminobenzol und/oder 2,3-Dimethyl-1,4-diaminobenzol und/oder 2,6-Diethyl-1,4-diaminobenzol enthält, wobei der genannte Bestandteil (A) kein Jodidion in einer zum Oxidieren der Oxidationsfarbstoff-Vorstufe ausreichenden Menge aufweist, und
- einen Bestandteil (B) aus einer Zusammensetzung umfaßt, die in einem zur Färbung geeigneten Milieu ein oxidierendes Agens enthält, und daß man vor der Aufbringung die Zusammensetzungen (A) und (B) in Mengenanteilen von 10 bis 90% für den Bestandteil (A) und von 90 bis 10% für den Bestandteil (B) vermischt, und zwar so, daß man eine Zusammensetzung erhält, die einen pH-Wert von weniger als 7 aufweist, und daß man diese Mischung sofort nach ihrer Zubereitung auf die keratinischen Fasern aufbringt, nach der Aufbringung eine Verweilzeit folgen läßt und gegebenenfalls abschließend spült und dann schamponiert.

11. Verfahren zur Färbung gemäß jedem der Ansprüche 1 bis 9,
dadurch **gekennzeichnet,** daß
man auf die Haare die Zusammensetzung aufbringt und sie 3 bis 40 Minuten lang veweilen läßt, die Haare spült und sie gegebenenfalls vor einer erneuten Spülung schamponiert und dann trocknet.